# EUROPEAN PATENT APPLICATION

(11) **EP 0 866 046 A1**
(43) Date of publication of application: **23.09.1998**
(21) Application number: 98302069.4
(22) Date of filing: 19.03.1998
(51) Int. Cl.: C07C 17/12

(54) **Catalytic process for selective aromatic bromination**

(30) Priority: 20.03.1997 GB 9705800
(71) Applicant: CONTRACT CHEMICALS LIMITED, Prescot, Merseyside L34 9HY (GB)
(72) Inventor: Bastock, Tony William, Sandbach Cheshire (GB); Trenbirth, Brian, Haslington Crewe (GB); Clark, James Hanley, York (GB); Ross, Joanne, New Milton Hampshire (GB)
(74) Representative: W.P. Thompson & Co.

(57) **Abstract**

There is disclosed a process for the bromination of an aromatic compound, which comprises treating the aromatic compound with elemental bromine in the presence of a catalyst comprising a zinc salt, preferably a halide salt, adsorbed on an inert support.

## Description

The present invention relates to a catalytic process for selective aromatic bromination.

Many processes are known for the bromination of mono-substituted aromatic compounds. Traditionally Lewis acids such as iron (III) bromide or aluminium (III) bromide are used. However, there are many disadvantages with such processes, such as the formation of by-products as a result of side reactions, and the lack of selectivity to the para-isomer. All result in low yields and can lead to unacceptable environmental effects. Other problems can be associated with recycling or disposing of expensive, possibly toxic or harmful, catalytic wastes.

There are three main types of reagent used for bromination reactions : N-bromosuccinimide, hydrobromic acid and elemental bromine. The use of elemental bromine for selective bromination reactions has been studied extensively and is the source of bromine used in this process.

Initial studies into the bromination of activated mono-substituted benzenes used traditional Friedel-Crafts Lewis acid catalysts. A review of such reactions is given in George A. Olah's book "Friedel-Crafts and Related Reactions" Volume III Part 2 pp 1518-1593. These Lewis acids form complexes with the bromine producing a source of Br⁺. Unfortunately such catalysts result in low selectivity to the para-isomer and has lead to the investigation into different catalysts.

More recently, most investigations have resulted in the use of zeolites as highly selective brominating catalysts. Several papers have been published, including work by Wortel et al., Journal of Catalysis., 60., 110-120 (1979); J. Zabicky et al., Zeolites., 7,499-502 (1989); F. de la Vega and Y. Sasson., zeolites., 13., 341-347 (1993) and K. Smith et al., J. Chem. Soc., Chem. Commun., 467-468 (1996). There is also an excellent review covering general halogenations using zeolites by P Ratnasamy et al, Applied Catalysis A: General., 135., 25-55 (1996). However, the main disadvantage of such processes is the vast quantity of catalyst required to achieve high selectivity.

The present invention seeks to provide a catalytic process for highly selective bromination of aromatic compounds.

According to the present invention there is provided a process for the bromination of an aromatic compound, which comprises treating the aromatic compound with elemental bromine in the presence of a catalyst comprising a zinc salt adsorbed on an inert support.

The salt is preferably in the form of a halide, particularly a bromide, though a chloride salt can be used.

Suitable inert supports include silica, alumina, and acid activated clay (eg K10). The best selectivity to the para-isomer was obtained when using silica.

The catalyst is preferably activated eg by heating to a temperature of 150-300°C.

The catalyst preferably comprises 0.1 to 3.0 mmol of the salt per gram of inert support.

The reaction is prererably carried out in the presence of a solvent. Various solvents may be used in this process including carbon tetrachloride, chloroform, dichloromethane, hexane, and cyclohexane. The best selectivity to the para-isomer was obtained when using dichloromethane or hexane.

This process is preferably carried out in the dark so as to minimise the production of bromine radicals as this would result in reduced selectivity to the desired product.

Suitable aromatic compounds which may be brominated by the process of the present invention include those compounds having the general formulae: 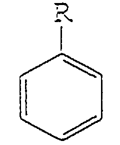 Wherein R may be H, halogen, substituted or unsubstituted, primary, secondary and tertiary alkyl, alkyl, haloalkyl, substituted or unsubstituted alkoxy, hydroxy, substituted or unsubstituted primary, secondary or tertiary amino, substituted or unsubstituted mercapto, substituted or unsubstituted amido or substituted or unsubstituted aryl.

In a preferred embodiment of the process of this invention the aromatic compound to be brominated is mixed with the catalyst in a suitable solvent. Bromine is added and the mixture is stirred at a desired temperature, the reaction is continued for an appropriate time and the catalyst is then separated by filtration, centrifugation, decantation or a similar technique.

In contrast with many known processes the catalyst may be re-used.

The bromoaromatic product can then be isolated and purified by standard, known techniques.

The present invention will now be further described with reference to, but is in no manner limited to, the following examples.

### Example 1

A catalyst was prepared as follows:- ZnBr₂ (1.24 g, 5 mmol) was dissolved in methanol (50 ml) and a commercial acid treated clay, K-10 (5 g) was added, and the mixture stirred for 1 hour. The methanol was then removed by rotary evaporation and the remaining fine powder activated at 200°C. This resulted in a catalyst with a loading of 1 mmol/g ZnBr₂ on K-10.

0.6g of the catalyst (1 mmol/g ZnBr₂ on K-10) was slurrried in hexane (15 ml) and toluene (1 ml, 9.4 mmol). Bromine (0.48 ml, 9.4 mmol) was added, and the reaction stirred at room temperature in the dark.

Analysis of the reaction mixture by GC showed 100% conversion of toluene to 67.4% p-bromotoluene, 27.8% o-bromotoluene and 4.8% dibromotoluene within 2 minutes.

In comparison with the non-catalysed reaction, which on analysis by GC resulted in only a 0.8% conversion of toluene to 66.6% p-bromotoluene and 33.3% o-bromotoluene within 2 minutes.

### Example 2

A catalyst was prepared as follows:- ZnBr₂ (2.17 g, 8.75 mmol) was dissolved in methanol (50 ml). Kieselgel 100 silica (100Å silica) (5g) was added, and the mixture stirred for 1 hour. The methanol was then removed by rotary evaporation and the remaining fine powder activated at 200°C. This resulted in a catalyst with a loading of 1.75 mmol/g ZnBr₂ on silica.

0.6g of the catalyst (1.75 mmol/g ZnBr₂ on silica (100Å)) was slurried in hexane (15 ml) and toluene (1 ml, 9.4 mmol). Bromine (0.48 ml, 9.4 mmol) was added, and the reaction stirred at room temperature in the dark.

Analysis of the reaction mixture by GC showed 100% conversion of toluene to 65.1% p-bromotoluene, 24.2% o-bromotoluene and 10.6% dibromotoluene within 2 minutes.

### Example 3

0.6g of the catalyst prepared in Example 1 (1 mmol/g ZnBr₂ on K-10) was slurried in hexane (15 ml) and ethylbenzene (1 ml, 8.2 mmol). Bromine (0.42 ml, 8.2 mmol) was added, and the reaction stirred at room temperature in the dark.

Analysis of the reaction mixture by GC showed 100% conversion of ethylbenzene to 75.4% p-bromoethylbenzene and 24.6% o-bromoethylbenzene within 5 minutes.

In comparison with the non-catalysed reaction, which on analysis by GC resulted in a 0% conversion of ethylbenzene within 5 minutes.

### Example 4

0.6g of the catalyst prepared in Example 2 (1.75 mmol/g ZnBr₂ on silica (100Å)) was slurried in hexane (15 ml) and ethylbenzene (1 ml, 8.2 mmol). Bromine (0.42 ml, 8.2 mmol) was added, and the reaction stirred at room temperature in the dark.

Analysis of the reaction mixture by GC showed 68.5% conversion of ethylbenzene to 70.4% p-bromoethylbenzene and 29.6% o-bromoethylbenzene within 5 minutes.

### Example 5

0.6g of the catalyst prepared in Example 1 (1 mmol/g ZnBr₂ on K-10) was slurried in hexane (15 ml) and t-butylbenzene (1-ml, 6.5 mmol). Bromine (0.33 ml, 6.5 mmol) was added, and the reaction stirred at room temperature in the dark.

Analysis of the reaction mixture by GC showed 100% conversion of t-butylbenzene to 100% p-bromo t-butylbenzene within 2 minutes.

In comparison with the non-catalysed reaction, which on analysis by GC resulted in a 0% conversion of t-butylbenzene within 2 minutes.

### Example 6

0.6g of the catalyst prepared in Example 2 (1.75 mmol/g ZnBr₂ on silica (100Å)) was slurried in hexane (15 ml) and t-butylbenzene (1 ml, 6.5 mmol). Bromine (0.33 ml, 6.5 mmol) was added, and the reaction stirred at room temperature in the dark.

Analysis of the reaction mixture by GC showed 100% conversion of t-butylbenzene to 100% p-bromo-t-butylbenzene within 2 minutes.

### Example 7

0.6g of the catalyst prepared in Example 1 (1 mmol/g ZnBr₂ on K-10) was slurried in hexane (15 ml) and bromobenzene (1 ml, 9 mmol). Bromine (0.76 ml, 15 mmol) was added, and the reaction stirred at room temperature in the dark.

Analysis of the reaction mixture by GC showed 100% conversion of bromobenzene to 89.5%.p-dibromobenzene and 10.5% o-dibromobenzene within 3 hours.

In comparison with the non-catalysed reaction, which on analysis by GC resulted in only a 10% conversion of bromobenzene to 80.4% p-dibromobenzene and 19.6% o-dibromobenzene within 24 hours.

### Example 8

0.6g of the catalyst prepared in Example 2 (1.75 mmol/g ZnBr₂ on silica (100Å)) was slurried in hexane (15 ml) and bromobenzene (1 ml, 9 mmol). Bromine (0.76 ml, 15 mmol) was added, and the reaction stirred at room temperature in the dark.

Analysis of the reaction mixture by GC showed 100% conversion of bromobenzene to 90% p-dibromobenzene and 10% o-dibromobenzene within 1.5 hours.

### Example 9

0.6g of the catalyst prepared in Example 1 (1 mmol/g ZnBr₂ on K-10) was slurried in dichloromethane (15 ml) and bromobenzene (1 ml, 9 mmol). Bromine (0.76 ml, 15 mmol) was added, and the reaction stirred at room temperature in the dark.

Analysis of the reaction mixture by GC showed 93.5% conversion of bromobenzene to 91.7% p-dibromobenzene and 8.3% o-dibromobenzene within 7 hours.

### Example 10

0.6g of the catalyst prepared in Example 2 (1.75 mmol/g ZnBr₂ on Silica (100Å)) was slurried in hexane (15 ml) and fluorobenzene (lml, 10 mmol). Bromine (0.52ml, 10 mmol) was added, and the reaction stirred at room temperature in the dark. Analysis of the reaction mixture by GC showed 78.9% conversion of fluorobenzene to 98.9% p-bromofluorobenzene and 1.1% o-bromofluorobenzene within 15 minutes. In comparison with the non-catalysed reaction which on analysis by GC resulted in 0% conversion of fluorobenzene within 2 minutes.

## Claims

1. A process for the bromination of an aromatic compound, which comprises treating the aromatic compound with elemental bromine in the presence of a catalyst comprising a zinc salt adsorbed on an inert support.

2. A process as claimed in claim 1, in which the salt is in the form of a halide.

3. A process as claimed in claim 1 or 2, in which the inert support is silica, alumina or acid activated clay.

4. A process as claimed in any one of the preceding claims, in which the catalyst has been activated at a temperature of 150-300°C.

5. A process as claimed in any one of the preceding claims, which is carried out in the presence of a solvent.

6. A process as claimed in any one of the preceding claims, which is carried out in the dark.

7. A process as claimed in any one of the preceding claims, in which the aromatic compound to be brominated has the general formulae: 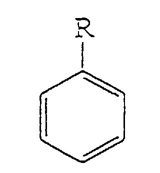 wherein R is H, halogen, substituted or unsubstituted primary, secondary and tertiary alkyl, haloalkyl, substituted or unsubstituted alkoxy, hydroxy, substituted or unsubstituted primary, secondary or tertiary amino, substituted or unsubstituted mercapto, substituted or unsubstituted amido or substituted or unsubstituted aryl.

8. A process as claimed in any one of the preceding claims, in which the aromatic compound to be brominated is mixed with the catalyst in a suitable solvent, bromine is added and the mixture is stirred the catalyst is then separated by filtration, centrifugation, decantation or a similar technique, and the bromoaromatic product is isolated and purified.

9. A process as claimed in any one of the preceding claims, in which the catalyst comprises 0.1 to 3.0 mmol of the salt per gram of inert support.
